# EUROPÄISCHE PATENTSCHRIFT

(11) **EP 0 554 565 B1**
(45) Veröffentlichungstag und Bekanntmachung des Hinweises auf die Patenterteilung: **05.08.1998**
(21) Anmeldenummer: 92122001.8
(22) Anmeldetag: 24.12.1992
(51) Int. Cl.: A61F 13/15

(54) **Saugfähige hygienische Einlage zum Schutz der Unterwäsche**
Adsorbent, hygienic liner for underwear protection
Protège-slip absorbant et hygiénique

(30) Priorität: 01.02.1992 DE 9201239 U
(43) Veröffentlichungstag der Anmeldung: 11.08.1993
(73) Patentinhaber: Kimberly-Clark GmbH, 56070 Koblenz (DE)
(72) Erfinder: Raidel, Maria, Dr. Dipl.-Chem., W-8507 Oberasbach (DE); Hübner, Peter, W-8501 Winkelhaid (DE); Petranyi, Pal, Dr. Dipl.-Chem., W-8500 Nürnberg 90 (DE)
(74) Vertreter: Leidescher, Thomas, Dr.

(56) Entgegenhaltungen:
- WO-A-82/03170
- WO-A-92/07536
- GB-A- 2 244 653
- US-A- 4 808 178

## Beschreibung

Die Erfindung betrifft eine saugfähige hygienische Einlage zum Schutz der Unterwäsche, wie Damenbinde, Slipeinlage oder superdünne Binde mit
- einem Saugkörper,
- einer flüssigkeitsdurchlässigen oberen Abdeckung
- und einer flüssigkeitsdichten unteren Abdeckung.

Derartige Einlagen sind seit langem bekannt. Die in ihnen vorhandenen Saugkörper bestehen heute meist aus trocken abgelegten Zellstoff-Flocken, welche in manchen Fällen noch mit Quellstoffen vermischt sind. Bei sehr dünnen Einlagen dieser Art, den sogenannten superdünnen Binden, wird auf einen Saugkörper aus Zellstoff-Flocken verzichtet und an dessen Stelle tritt ein Band aus sogenanntem Airlaid-Material, das sind trocken im Luftstrom abgelegte Zellstoff-Flocken, welche mit einem thermoplastischen Bindemittel in Form von Fasern oder Partikeln vermischt sind, wobei das Bindemittel nach dem Ablegen durch eine Wärmebehandlung verflüssigt und die Zellstoff-Flocken auf diese Weise zu einem zusammenhängenden Band miteinander verbunden werden. Superdünne Binden dieser Art haben im allgemeinen eine Gesamtstärke von etwa 3 bis 5 mm.

Die flüssigkeitsdurchlässige obere Abdeckung besteht im allgemeinen aus Vliesstoffen, das sind dünne Lagen aus Baumwoll- oder Zellstoff- bzw. Zellwoll-Fasern, in manchen Fällen auch aus Kunststoff-Fasern, welche mit einem thermoplastischen Bindemittel oder mit sich selbst thermoverfestigt sind. Anstelle derartiger Vliesstoffe können auch gelochte thermoplastische Folien treten, wobei die Lochform sowie eine zusätzliche Prägung der Folie ein textilartiges Verhalten vermittelt.

Die flüssigkeitsdichte untere Abdeckung besteht im allgemeinen aus einer Polyethylenfolie, die gegebenenfalls noch mit einer Tissue-Lage oder einem ähnlichen Material kaschiert sein kann.

Die vorstehend beschriebenen Produkte gehören seit langem zum Stande der Technik. Zum Stande der Technik gehören auch Weiterentwicklungen, bei denen die hygienischen Einlagen mit seitlichen flügelähnlichen Streifen versehen sind, welche den Zweck haben, die Einlage in der Unterwäsche sicher und unverrutschbar festzuhalten. Die Halteflügel werden zu diesem Zweck, nachdem die Einlage in ein Unterwäschestück, beispielsweise ein Höschen eingelegt ist, nach unten umgebogen, durch das Beinloch hindurchgeführt und an der Unterseite des Steges zwischen den beiden Beinlöchern festgeklebt. Binden, auch Slipeinlagen dieser Form sind beispielsweise in der europäischen Patentanmeldung 0 249 924 oder auch der europäischen Patentanmeldung 0 301 491 beschrieben. Daneben wird in der englischen Patentanmeldung GB-A-2 244 653 eine hygienische Einlage beschrieben, die mit seitlichen flügelähnlichen Streifen versehen ist. Dieser absorbierende Artikel ist mit einer flüssigkeitsdurchlässigen oberen Abdeckung, einer flüssigkeitsdichten unteren Abdeckung und einem dazwischen angeordneten absorbierenden Material ausgestattet. Die flügelähnlichen Streifen können als ein Teil der flüssigkeitsundurchlässigen Schicht, als ein Teil der oberen Abdeckung verbunden mit der flüssigkeitsundurchlässigen Schicht, oder als eigenständige Teile ausgebildet sein. In der Patentschrift US-A-4 808 178 wird ein absorbierender Artikel mit einem flüssigkeitsabsorbierenden Kern beschrieben, der von einer äußeren Abdeckschicht eingefaßt ist. An dieser äußeren Abdeckschicht sind Kragen befestigt, die jeweils ein von diesem fixen Befestigungspunkt beabstandetes elastisches freies Ende aufweisen.

Bei saugfähigen hygienischen Einlagen zum Schutz der Unterwäsche kommt es wesentlich darauf an, daß das Saugvermögen sowie das Flüssigkeitsaufnahmevermögen möglichst groß sind, und daß überhaupt die gesamte Konstruktion der Einlage so ist, daß auch bei starkem Flüssigkeitsanfall das Unterwäschestück sicher vor Verschmutzung geschützt wird.

Der Erfindung liegt die Aufgabe zugrunde, die vorbekannten hygienischen Einlagen weiter zu verbessern, ihre Herstellung zu vereinfachen, aber insbesondere ihre Sicherheit gegenüber Flüssigkeitsaustritt zu erhöhen.

Zur Lösung dieser Aufgabe wird von einer saugfähigen hygienischen Einlage ausgegangen, die folgende aus dem Stand der Technik bekannten Merkmale aufweist:
- die obere Abdeckung bedeckt die Oberseite sowie wenigstens teilweise die beiden Seitenflächen des Saugkörpers;
- die untere Abdeckung bedeckt die Unterseite sowie die beiden Seitenflächen des Saugkörpers mitsamt den dort befindlichen Teilen der oberen Abdeckung und reicht bis über zwei Längsrandstreifen auf der Oberseite des Saugkörpers;
- die Seitenstreifen der oberen Abdeckung sind
   -- entweder mit den Seitenstreifen der unteren Abdeckung verklebt oder
   -- bis unter die Unterseite des Saugkörpers geführt;
- die untere Abdeckung ist im Bereich der auf der Oberseite des Saugkörpers verlaufenden Randstreifen nach außen umgeschlagen und bildet dort einen Kragen.

Zur Lösung der Aufgabe werden diese Merkmale mit dem Kennzeichnungsteil des Anspruches 1 kombiniert und somit eine saugfähige hygienische Einlage vorgeschlagen, die eine untere Abdeckung aufweist, die im Bereich der Längsrandstreifen jeweils an ihren beiden Endbereichen mit der Saugkörperoberfläche und/oder der oberen Abdeckung verklebt ist und daneben in dem dazwischen liegenden, nicht verklebten Bereich je eine zusätzliche Rinne Flüssigkeitseinlauf bildet.

Durch die Kombination dieser Merkmale werden folgende Vorteile erzielt: Zum einen wird eine sichere Abdichtung des sonst gegen Überlauf sehr gefährdeten Randstreifens des Saugkörpers erzielt. Zum anderen wird aber auch erreicht, daß die gesamte Einlage Wannenform annimmt, wodurch insbesondere bei starkem plötzlichen Flüssigkeitsanfall diese Flüssigkeit aufgefangen und über eine maximale Oberfläche des Saugkörpers verteilt wird. Es entsteht auf diese Weise eine Einlage mit optimiertem Ansaugverhalten bei gleichzeitig hohem Flüssigkeitsaufnahme- und -rückhaltevermögen.

Die vorgeschlagene Konstruktion einer saugfähigen hygienischen Einlage bietet außerdem den Vorteil, daß sie in mehreren Variationen leicht herstellbar ist und nach Art eines Baukastens je nach Wunsch zu unterschiedlichen Formen ausgebaut werden kann. So entsteht zunächst nach der vorstehend aufgeführten Merkmalskombination ein Basiskörper, der schon alle wesentlichen Eigenschaften der verbesserten hygienischen Einlage aufweist. Sein Kragen sorgt für eine gute Anlage am Körper der Benutzerin. Der Kragen bietet andererseits den Vorteil, daß seitlich abstehende Befestigungsflügel, wie sie einleitend erwähnt wurden, leicht aufgeklebt werden können. Falls dies erwünscht ist, kann der Kragen im Bereich der Längsmitte durch querabstehende, stummelförmige Ansätze etwas erweitert sein, so daß dort eine vergrößerte Fläche für die Aufnahme der Befestigungsflügel entsteht.

Gemäß einer bevorzugten Ausführungsform der erfindungsgemäßen Einlage ist die untere Abdeckung im Bereich zumindest der Längsrandstreifen über die volle Länge der Einlage mit der Saugkörperoberfläche und/oder der oberen Abdeckung verklebt. Vorzugsweise kann sich diese Verklebung auch über die die Seitenflächen des Saugkörpers überdeckenden Seitenstreifen der oberen und unteren Abdeckung erstrecken. Durch diese Maßnahmen wird eine besonders kompakte und in sich formstabile Ausbildung der Einlage erreicht, wobei gleichzeitig ein außerordentlich gutes Rückhaltevermögen für Körperflüssigkeiten aufgrund der durch die Verklebung stabilisierten Wannenform der Einlage erzielt wird.

Gemäß einer alternativen Ausführungsform der erfindungsgemäßen Einlage ist vorgesehen, daß die untere Abdeckung im Bereich der Längsrandstreifen jeweils nur an ihren beiden Endbereichen mit der Saugkörperoberfläche und/oder der oberen Abdeckung verliebt ist und in dem dazwischen liegenden nicht verklebten Bereich je eine zusätzliche Rinne zum Flüssigkeitseinlauf bildet. Diese Rinne stellt praktisch ein zusätzliches Reservoir zur Aufnahme von Körperflüssigkeit dar, das insbesondere dann zum Einsatz kommt, wenn die Flüssigkeitsaufnahmekapazität des Saugkörpers erschöpft ist. Durch die seitlich hochgeführten Bereiche der unteren Abdeckung ist dabei eine sichere Rückhaltung der sich in der Rinne ansammelnden Flüssigkeit gewährleistet.

Zum weiteren Ausbau des Systems wird vorgeschlagen, daß der Kragen im Bereich der Längsmitte beidseitig zu quer abstehenden Befestigungsflügeln erweitert ist. Es entsteht auf diese Weise eine symmetrische Flügelbinde, wie sie grundsätzlich bereits bekannt ist.

Abweichend davon wird aber auch vorgeschlagen, daß der Kragen im Bereich der Längsmitte nur einseitig zu einem quer abstehenden Befestigungsflügel erweitert ist. Diese Ausführungsform ist besonders materialsparend und kann dennoch äußerst leicht und rutschsicher im Wäschestück befestigt werden. Zudem ist es für die Benutzerin leichter, nur einen quer abstehenden Flügel im Wäschestück zu befestigen als deren zwei. Die Anordnung gestattet es, bequemer die hygienische Einlage faltenfrei und ohne Querspannungen anzulegen. Die Befestigungsflügel in jeglicher Konfiguration können dabei einstückig mit der unteren Abdeckung ausgebildet oder auf den Kragen bzw. dessen stummelförmige Ansätze aufgeliebt sein.

In weiterer Ausgestaltung der Erfindung wird vorgeschlagen, daß an der Unterseite der Einlage ein sich in Längsrichtung erstreckender Haftstreifen und zusätzlich an den Befestigungsflügeln je eine weitere Haftstelle angeordnet ist. Schließlich hat es sich aber auch als vorteilhaft erwiesen, wenn an der Unterseite der Einlage ein sich in Querrichtung erstreckender Haftstreifen angeordnet ist, der sich bis zu den Enden der Befestigungsflügel erstreckt. Bei dieser Anordnung kann auf einen weiteren Streifen in Längsrichtung verzichtet werden. Vorteilhaft ist es dabei, wenn anstelle eines durchgehenden Haftstreifens eine Folge kleinerer Haftetiketten angeordnet wird.

In weiterer Ausgestaltung der Erfindung wird außerdem vorgeschlagen, daß die flüssigkeitsdichte untere Abdeckung zumindest im Bereich ihrer körperzugewandten Oberseite seitlich außerhalb des den Kragen bildenden Umschlages mit einem hydrophoben Vliesstoff z.B. einem Polypropylen-Spunbond-Vliesstoff beschichtet ist. Durch den hydrophoben Charakter der einen angenehmen Griff mit sich bringenden Vliesstoffbeschichtung wird vermieden, daß diese nennenswerte Transporteigenschaften für Körperflüssigkeiten zeigt, so daß ein Benetzen der seitlichen Teile der unteren Abdeckung und der daraus gebildeten bzw. an sie angesetzten Befestigungsflügel verhindert wird. Dem selben Zweck dient die gemäß einer bevorzugten Ausführungsform der Erfindung vorgesehene Beschichtung auf der Innenseite der flüssigkeitsdichten unteren Abdeckung zumindest in dem die beiden Seitenflächen des Saugkörpers umgreifenden Bereich in Form eines in Längsrichtung verlaufenden, aufgedruckten Streifens einer vorzugsweise stark hydrophoben Druckfarbe. Damit wird sichergestellt, daß aus diesen Bereichen praktisch keine Körperflüssigkeit zur Oberseite der Einlage hin transportiert wird.

In einer weiteren bevorzugten Ausführungsform der Erfindung ist es vorgesehen, die Befestigungsflügel als ein- oder zweistückige Zuschnitte auf die körperabgewandte Unterseite der unteren Abdeckung aufzukleben. Durch diese Ausgestaltung werden die Funktionen einerseits der Flüssigkeitsrückhaltung und andererseits der Befestigung der Einlage in einem Wäschestück von zwei getrennten Teilen der Einlage erfüllt. Insofern kann jeder dieser entsprechenden Teile optimal an seinen Einsatzzweck angepaßt werden. Auch hier spiegelt sich der Systemgedanke wieder, wonach aufbauend auf den in Anspruch 1 angegebenen Basiskörper verschiedene Konfigurationen hinsichtlich der Befestigungsflügel, Haftstreifen, Rinnenausbildung und dergleichen geschaffen werden können.

Die Erfindung wird im folgenden anhand der beigefügten Zeichnung näher erläutert. Es stellen dar:
- Fig. 1: eine perspektivische Ansicht einer Ausführungsform der Erfindung in Form des Basiskörpers;
- Fig. 2 bis 5: Querschnitte durch den Basiskörper gemäß Fig. 1 entlang der dort eingezeichneten Schnittlinien II-II bis V-V in unterschiedlichen Ausführungsformen;
- Fig. 6 bis 8: Draufsichten auf die Unterseiten verschiedener Ausführungsformen des ausgebauten Systems und
- Fig. 9: einen Querschnitt durch eine Einlage in einer weiteren Ausführungsform.

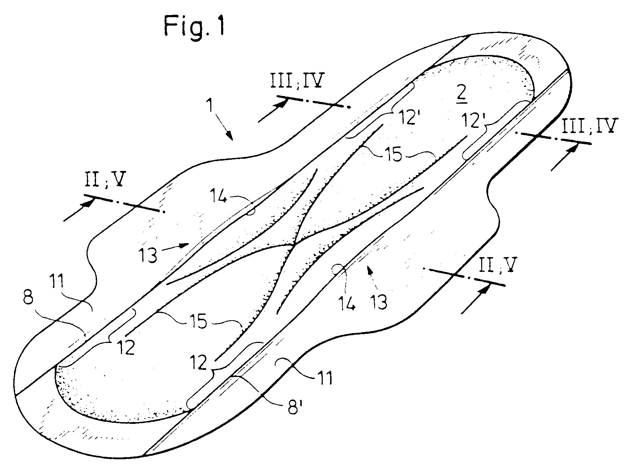

Die in den Fig. 1 bis 9 in unterschiedlichen Ausführungsformen dargestellte saugfähige hygienische Einlage ist als Ganzes mit 1 bezeichnet. Sie besteht grundsätzlich aus einem Saugkörper 2, einer flüssigkeitsdurchlässigen oberen Abdeckung 3 und einer flüssigkeitsdichten unteren Abdeckung 4.

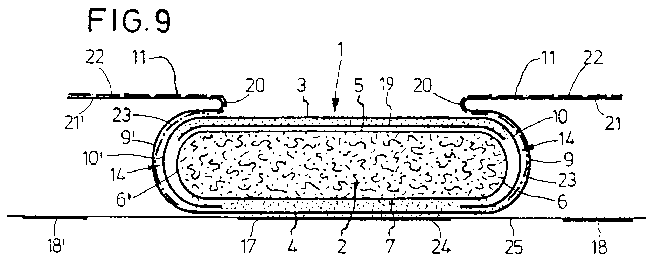

Die obere Abdeckung 3 bedeckt die Oberseite 5 des Saugkörpers 2 sowie wenigstens teilweise die beiden Seitenflächen 6 und 6' dieses Saugkörpers. Zwischen der Oberseite des Saugkörpers 2 und der oberen Abdeckung 3 ist eine flüssigkeitsdurchlässige Zwischenschicht 19 (Fig. 9) vorgesehen, die vollflächig mit der oberen Abdeckung 3 verleimt ist. Bei einer entsprechend stabilen Ausbildung der oberen Abdeckung 3 kann dabei auf die Zwischenschicht 19 verzichtet werden. Die untere Abdeckung 4 bedeckt die Unterseite 7 sowie die beiden Seitenflächen 6 und 6' des Saugkörpers 2 mitsamt den dort befindlichen Teilen der oberen Abdeckung 3. Die untere Abdeckung 4 reicht bis über zwei Längsrandstreifen 8 und 8', die sich auf der Oberseite des Saugkörpers 2 befinden.

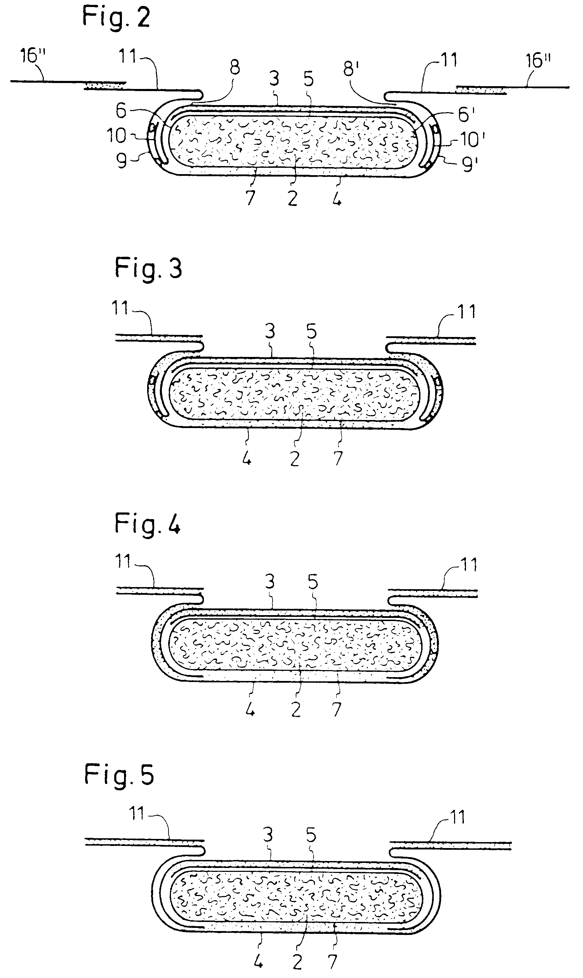

Die Seitenstreifen 10 und 10' der oberen Abdeckung 3 sind entweder mit den Seitenstreifen 9 und 9' der unteren Abdeckung 4 verliebt, wie dies in den Fig. 2 und 3 dargestellt ist oder sie sind bis unter die Unterseite 7 des Saugkörpers 2 geführt, wie dies in den Fig. 4 und 5 dargestellt ist.

Die untere Abdeckung 4 ist im Bereich der Randstreifen 8, 8', die auf der Oberseite des Saugkörpers verlaufen, nach außen umgeschlagen (Umschlag 20) und bildet einen Kragen 11. Die Randstreifen 8, 8' sind jeweils an ihren beiden Endbereichen 12 und 12' mit der Saugkörperoberfläche und/oder der oberen Abdeckung 3 verklebt und bilden in dem dazwischen liegenden, nicht verklebten Bereich 13 je eine zusätzliche Rinne 14 zum Flüssigkeitseinlauf. Statt einen nicht verklebten Bereich 13 vorzusehen, können die Randstreifen 8, 8' sowie die Seitenstreifen 9, 9' der unteren Abdeckung 4 über die gesamte Länge der Einlage mit der oberen Abdeckung 3 verklebt sein, so daß die zusätzliche Rinne 14 verschlossen ist. Für diesen Fall entsprechen die Schnitte gemäß den Fig. 2 und 5 im Rinnenbereich denen der Fig. 3 und 4.

Aus der in Fig. 1 ersichtlichen perspektivischen Darstellung geht auch hervor, daß die Oberfläche des Saugkörpers 2 eine zusätzliche Prägung in Form von Prägelinien 15 aufweist, die ebenfalls der Vergrößerung der Oberfläche dient und folglich das Flüssigkeitsaufnahmevermögen erhöht, die aber auch dekorativen Charakter hat.

Weiterhin geht aus Fig. 1 hervor, daß der seitliche Kragen 11 im Bereich der Längsmitte der Einlage mit einstückig angeformten, quer abstehenden, stummelförmigen Ansätzen 21, 21' versehen ist. Auf diese stummelförmigen Ansätze können Befestigungsflügel 16'' aufgeklebt werden, wie dies in Fig. 2 gezeigt ist. Die Verklebung zwischen den Ansätzen 21, 21' und den Befestigungsflügeln 16'' ist dabei durch eine Punktierung angedeutet.

Entsprechende Punktierungen sind in allen Schnitten gemäß den Fig. 2 bis 5 zur Darstellung von Verklebungen zwischen entsprechenden Teilen der Einlage verwendet. Im übrigen sind seitlich aufgesetzte Befestigungsflügel in den Fig. 3 bis 5 weggelassen.

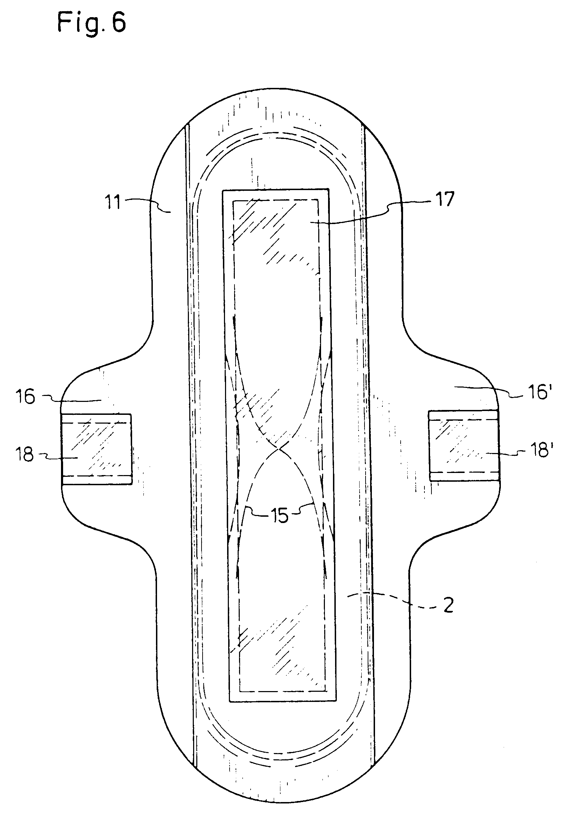

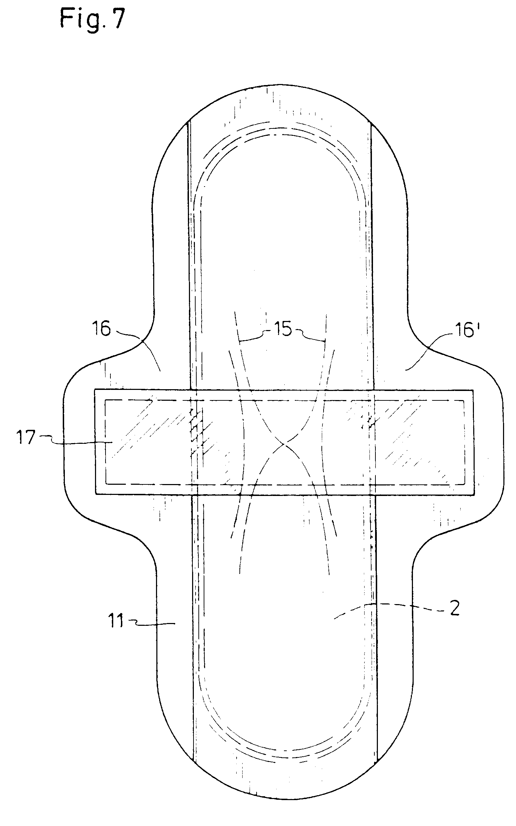

Bei den Ausführungsformen gemäß den Fig. 6 und 7 ist der Kragen 11 im Bereich der Längsmitte beidseitig zu quer abstehenden Befestigungsflügeln 16 und 16' erweitert. Bei der Ausführungsform gemäß Fig. 6 befindet sich an der Unterseite der Einlage in an sich bekannter Weise ein Haftstreifen 17, der sich in Längsrichtung der Einlage erstreckt. Zusätzlich ist an den Befestigungsflügeln 16 und 16' je eine weitere Haftstelle 18 und 18' angeordnet.

Eine andere interessante Verbesserung ist in Fig. 7 dargestellt. Dort erstreckt sich der Haftstreifen 17 quer zur Längsrichtung der Einlage, wodurch sich zusätzliche Haftstellen an den Befestigungsflügeln erübrigen.

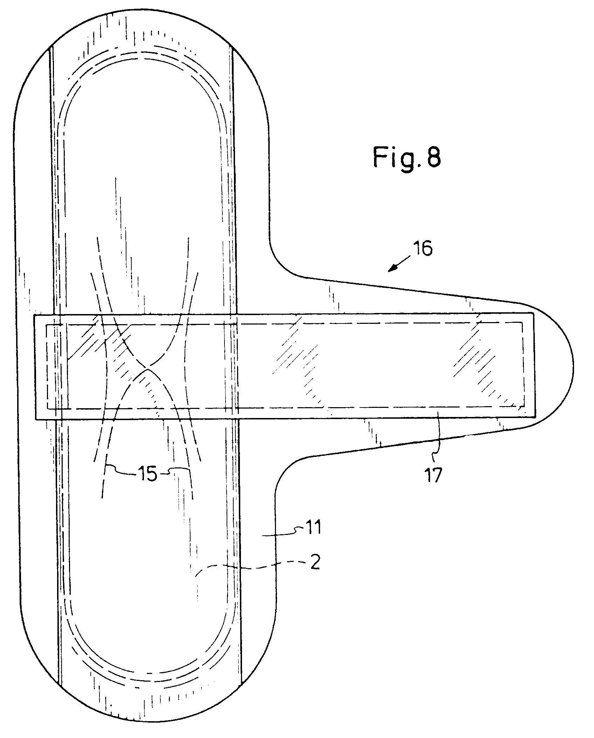

In Fig. 8 ist eine weitere interessante Ausführungsform der Erfindung wiedergegeben, bei der der Kragen 11 im Bereich der Längsmitte nur einseitig zu einem quer abstehenden Befestigungsflügel 16 erweitert ist. Auch bei dieser Ausführungsform ist ein Haftstreifen 17 vorhanden, der quer zur Längserstreckung angeordnet ist. Diese Ausführungsform zeichnet sich durch große Einfachheit der Handhabung, aber auch durch erhebliche Materialersparnis aus.

In Fig. 9 ist eine weitere alternative Ausgestaltung einer erfindungsgemäßen Einlage dargestellt. Da sich der grundsätzliche Aufbau nicht von den Einlagen gemäß Fig. 1 bis 8 unterscheidet, werden im folgenden nur die Unterschiede näher beschrieben.

So ist die Einlage im Bereich des Umschlages 20, des Kragens 11 und der längsmittig angeordneten, stummelförmigen Ansätze 21 der unteren Abdeckung 4 mit einer aufkaschierten Beschichtung 22 (strichliert dargestellt) aus hydrophoben Vliesstoff versehen. Für diese Beschichtung 22 kann beispielsweise ein Polypropylen-Spunbond-Vliesstoff verwendet werden, der von Hause aus hydrophob ist. Zu ergänzen ist, daß die Beschichtung 22 auch vollflächig über die gesamte Breite der unteren Abdeckung 4 verlaufen kann.

Weiterhin ist die flüssigkeitsdichte untere Abdeckung 4 in den die beiden Seitenflächen 6, 6' des Saugkörpers 2 umgreifenden Bereich - also im Bereich ihrer Seitenstreifen 9, 9' - innenseitig mit einem in Längsrichtung der Einlage verlaufenden, aufgedruckten Streifen 23 (strichpunktiert dargestellt) einer stark hydrophoben Druckfarbe versehen. Bei dieser Druckfarbe kann es sich beispielsweise um einen Trennlack der Firma Gebrüder Schmidt Druckfarben, Gaugrafenstraße 4 - 8, D - 6000 Frankfurt/Main, handeln, der unter der Bezeichnung "90 BK 134" vertrieben wird. Dieser Trennlack ist auf der Basis eines hochmolekularen, nicht reaktiven Kunstharzes in organischen Lösemitteln hergestellt und weist als Trenn- und Scheuerschutzmittel ein modifiziertes, inertes Wachs auf. Bei einer offenen Ausgestaltung der Rinne 14, wie dies in Fig. 9 dargestellt ist, ist der Streifen 23 damit im Bereich der Außenseite der Rinne 14 an den Seitenstreifen 9, 9' der unteren Abdeckung 4 vorgesehen. Im übrigen ist darauf hinzuweisen, daß der Druckstreifen 23 direkt auf die als dünne Polyethylenfolie ausgebildete untere Abdeckung 4 oder auf eine gegebenenfalls darauf aufgebrachte Beschichtung 22 oder zwischen eine solche Beschichtung 22 und der Folie der unteren Abdeckung 4 aufgedruckt sein kann.

Wie weiterhin aus Fig. 9 deutlich wird, ist an der körperabgewandten Unterseite 24 der unteren Abdeckung 4 ein einstückiger Zuschnitt 25 aufgeklebt, dessen beide über den Saugkörper 2 seitlich hinausstehenden Abschnitte wiederum Befestigungsflügel für die Einlage bilden. An diesem Zuschnitt 25 können wiederum ein zentraler Haftstreifen 17 sowie weitere Haftstellen 18, 18' zur Fixierung der Einlage in einem Wäschestück vorgesehen sein. Zu ergänzen ist, daß der seitliche Kragen 11 der Einlage gemäß Fig. 9 entweder - wie in dieser Figur dargestellt - unbefestigt seitlich abstehen oder entlang der Seitenstreifen 9, 9' der unteren Abdeckung 4 nach unten geführt und mit diesen Seitenstreifen 9, 9' verklebt sein kann.

## Patentansprüche

1. Saugfähige hygienische Einlage zum Schutz der Unterwäsche, wie Damenbinde, Slipeinlage oder superdünne Binde mit
- einem Saugkörper (2);
- einer flüssigkeitsdurchlässigen oberen Abdeckung (3),
- und einer flüssigkeitsdichten unteren Abdeckung (4),
wobei
- die obere Abdeckung (3) die Oberseite (5) sowie wenigstens teilweise die beiden Seitenflächen (6; 6') des Saugkörpers (2) bedeckt;
- die untere Abdeckung (4) die Unterseite (7) sowie die beiden Seitenflächen (6; 6') des Saugkörpers (2) mitsamt den dort befindlichen Teilen der oberen Abdeckung (3) bedeckt und bis über zwei Längsrandstreifen (8; 8') auf der
Oberseite des Saugkörpers (2) reicht;
- die Seitenstreifen (10; 10') der oberen Abdeckung (3)
entweder mit den Seitenstreifen (9; 9') der unteren Abdeckung 4 verklebt sind oder
bis unter die Unterseite (7) des Saugkörpers (2) geführt sind; und
- die untere Abdeckung (4) im Bereich des auf der Oberseite (5) des Saugkörpers (2) verlaufenden Randstreifens (8; 8') nach außen umgeschlagen ist und einen Kragen (11) bildet;
**dadurch gekennzeichnet**,
- daß die untere Abdeckung (4) im Bereich der Längsrandstreifen (8; 8') jeweils an ihren beiden Endbereichen (12; 12') mit der Saugkörperoberfläche und/oder der oberen Abdeckung (3) verklebt ist und in dem dazwischen liegenden, nicht verklebten Bereich (13) je eine zusätzliche Rinne (14) Flüssigkeitseinlauf bildet.

2. Hygienische Einlage nach Anspruch 1, dadurch gekennzeichnet, daß der Kragen (11) im Bereich der Längsmitte beidseitig zu quer abstehenden Befestigungsflügeln (16; 16') erweitert ist.

3. Hygienische Einlage nach Anspruch 1, dadurch gekennzeichnet, daß der Kragen (11) im Bereich der Längsmitte einseitig zu einem quer abstehenden Befestigungsflügeln (16) erweitert ist.

4. Hygienische Einlage nach Anspruch 1, dadurch gekennzeichnet, daß der Kragen (11) im Bereich der Längsmitte beidseitig mit quer abstehenden, stummelförmigen Ansätzen (21; 21') insbesondere zur Anbringung von Befestigungsflügeln (16'') versehen ist.

5. Hygienische Einlage nach einem der Ansprüche 2 bis 4, dadurch gekennzeichnet, daß die Befestigungsflügel (16; 16') auf die Oberseite des Kragens (11) bzw. der stummelförmigen Ansätze (21; 21') aufgeklebt sind.

6. Hygienische Einlage nach einem der Ansprüche 2 bis 5, dadurch gekennzeichnet, daß an der Unterseite der Einlage ein sich in Längsrichtung erstreckender Haftstreifen (17) und zusätzlich an den Befestigungsflügel (16; 16') eine weitere Haftstelle (18; 18') angeordnet ist.

7. Hygienische Einlage nach einem der Ansprüche 2 bis 5, dadurch gekennzeichnet, daß an der Unterseite der Einlage ein sich in Querrichtung erstreckender Haftstreifen (17) angeordnet ist, der sich bis zu den Enden der Befestigungsflügel (16; 16') erstreckt.

8. Hygienische Einlage nach Anspruch 7, dadurch gekennzeichnet, daß anstelle des Haftsreifens (17) eine Folge kleinerer Haftetiketten angeordnet ist.

9. Hygienische Einlage nach einem der Ansprüche 1 bis 8, dadurch gekennzeichnet, daß die flüssigkeitsdichte untere Abdeckung (4) zumindest im Bereich ihrer körperzugewandten Oberseite seitlich außerhalb des Umschlages (20) mit einem hydrophoben Vliesstoff beschichtet (Beschichtung 22) ist.

10. Hygienische Einlage nach einem der Ansprüche 1 bis 9, dadurch gekennzeichnet, daß die flüssigkeitsdichte untere Abdeckung (4) zumindest in dem die beiden Seitenflächen (6; 6') des Saugkörpers (2) umgreifenden Bereich innenseitig mit einem in Längsrichtung verlaufenden, aufgedruckten Streifen (23) einer vorzugsweise stark hydrophoben Druckfarbe versehen ist.

11. Hygienische Einlage nach einem der Ansprüche 1, 4 oder 6 bis 10, dadurch gekennzeichnet, daß Befestigungsflügel als ein- oder zweistückige Zuschnitte (25) auf die körperabgewandte Unterseite (24) der unteren Abdeckung (4) aufgeklebt sind.

## Claims

1. Absorbent sanitary pad for the protection of undergarment, such as a sanitary napkin, panty shield or ultra-thin sanitary napkin, comprising
- an absorbent body (2),
- a liquid-permeable upper cover (3),
- and a liquid-impermeable lower cover (4),
wherein
- the upper cover (3) covers the top surface (5) as well as at least partially the two side surfaces (6;6') of the absorbent body (2);
- the lower cover (4) covers the bottom surface (7) and the two side surfaces (6; 6') of the absorbent body (2) as well as the parts of the upper cover (3) disposed thereon and extends over two longitudinal edge strips (8; 8') on the top surface of the absorbent body (2);
- the side strips (10;10') of the upper cover (3) are
- either adhesively attached to the side strips (9;9') of the lower cover (4) or
- extend below the bottom surface (7) of the absorbent body (2); and
- the lower cover (4) is folded over outwardly in the area of the edge strips (8; 8') extending on the top surface (5) of the absorbent body (2) and forms a collar (11);
**characterized in that**
- the lower cover (4) is adhesively attached to the surface of the absorbent body and/or the upper cover (3) in the area of the longitudinal edge strips (8; 8') at both end portions (12, 12') thereof, respectively, thus forming an additional channel (14), respectively, for receiving liquids in the intermediate portion (13) which is not adhesively attached.

2. The sanitary pad of claim 1, characterized in that the collar (11) is enlarged in the area of the longitudinal center on both sides to form transversely projecting attachment flaps (16;16').

3. The sanitary pad of claim 1, characterized in that the collar (11) is enlarged in the area of the longitudinal center on one side to form a transversely projecting attachment flap (16).

4. The sanitary pad of claim 1, characterized in that the collar (11) is provided in the area of the longitudinal center on both sides with transversely projecting, fin-shaped protrusions (21;21'), in particular for affixing attachment flaps (16'').

5. The sanitary pad of one of claims 2 to 4, characterized in that the attachment flaps (16;16') are adhered to the top surface of the collar (11) or the fin-shaped protrusions (21; 21').

6. The sanitary pad of one of claims 2 to 5, characterized in that an adhesion strip (17) extending in longitudinal direction of the pad is provided on the bottom surface of the pad and, in addition, a further adhesion spot (18;18') is provided on the attachment flap (16;16').

7. The sanitary pad of one of claims 2 to 5, characterized in that an adhesion strip (17) extending in transverse direction is provided on the bottom surface of the pad and extends up to the ends of the attachment flaps (16; 16').

8. The sanitary pad of claim 7, characterized in that a series of small-size adhesion labels are provided instead of the adhesion strip (17).

9. The sanitary pad of one of claims 1 to 8, characterized in that the liquid-impermeable lower cover (4) is laminated laterally outside of the fold (20) at least in the area of its top surface disposed toward the wearer's body with a hydrophobic nonwoven (coating 22).

10. The sanitary pad of one of claims 1 to 9, characterized in that the inside of the liquid-impermeable lower cover (4) is provided at least in the area engaging over the two side surfaces (6,6') of the absorbent body (2) with a printed strip (23) of a preferably highly hydrophobic print color extending in longitudinal direction.

11. The sanitary pad of one of claims 1, 4 or 6 to 10, characterized in that attachment flaps are adhesively attached as one-piece or two-piece cuttings (25) on the bottom surface (24) of the lower cover (4) disposed away from the wearer's body.

## Revendications

1. Garniture hygiénique absorbante pour la protection des sous-vêtements, telle que serviette hygiénique, protège-slip ou bande supermince présentant :
- un corps absorbant (2) ;
- une couverture supérieure (3) perméable aux liquides ; et
- une couverture inférieure (4) imperméable aux liquides ;
la couverture supérieure (3) recouvrant la face supérieure (5) ainsi qu'au moins en partie les deux faces latérales (6 ; 6') du corps absorbant (2) ;
- la couverture inférieure (4) recouvrant la face inférieure (7) ainsi que les deux faces latérales (6 ; 6') du corps absorbant (2), avec les parties s'y trouvant de la couverture supérieure (3), et s'étendant jusque sur deux bandes de bordure longitudinales (8 ; 8') de la face supérieure du corps absorbant (2) ;
- les bandes latérales (10 ; 10') de la couverture supérieure (3) étant soit collées aux bandes latérales (9 ; 9') de la couverture inférieure (4), soit guidées jusque sous la face inférieure (7) du corps absorbant (2) ; et
- la couverture inférieure (4) étant repliée vers l'extérieur, dans la région de la bande de bordure (8 ; 8') s'étendant sur la face supérieure (5) du corps absorbant (2), pour former une collerette (11) ;
caractérisée
- en ce que la couverture inférieure (4) est collée, dans la région des bandes de bordure longitudinales (8 ; 8'), sur chacune de ses deux zones d'extrémité (12 ; 12'), à la surface supérieure du corps absorbant et/ou à la couverture supérieure (3) et forme une rigole (14) supplémentaire d'arrivée de liquide, dans le zone (13) non collée qui est intercalée.

2. Garniture hygiénique selon la revendication 1, caractérisée en ce que la collerette (11) est élargie dans la zone médiane longitudinale, des deux côtés, en volets de fixation (16 ; 16') dépassant transversalement.

3. Garniture hygiénique selon la revendication 1, caractérisée en ce que la collerette (11) est élargie dans la zone médiane longitudinale, d'un côté, en un volet de fixation (16) dépassent transversalement.

4. Garniture hygiénique selon la revendication 1, caractérisée en ce que la collerette (11) est pourvue, dans la zone médiane longitudinale, des deux côtés, de prolongements (21 ; 21') en forme de tronçons, dépassent transversalement, en particulier pour l'application des volets de fixation (16'').

5. Garniture hygiénique selon l'une des revendications 2 à 4, caractérisée en ce que les volets de fixation (16 ; 16') sont collés sur la face supérieure de la collerette (11) ou des prolongements (21 ; 21') en forme de tronçons.

6. Garniture hygiénique selon l'une des revendications 2 à 5, caractérisée en ce que sur la face inférieure de la garniture est disposée une bande adhésive (17), s'étendant dans la direction longitudinale, et en ce qu'un autre emplacement adhésif (18 ; 18') est prévu au moins sur un volet de fixation (16 ; 16').

7. Garniture hygiénique selon l'une des revendications 2 a 5, caractérisée en ce que sur la face inférieure de la garniture est disposée une bande adhésive (17) s'étendant dans la direction transversale, qui s'étend jusqu'aux extrémités des volets de fixation (16 ; 16').

8. Garniture hygiénique selon la revendication 7, caractérisée en ce qu'une succession de petites étiquettes adhésives est prévue au lieu de la bande adhésive (17).

9. Garniture hygiénique selon l'une des revendications 1 à 8, caractérisée en ce que la couverture inférieure (4) imperméable aux liquides est revêtue d'un non-tissé hydrophobe (revêtement 22) se trouvant au moins dans la zone de sa face supérieure tournée vers le corps, sur les côtés, à l'extérieur du rabat (20).

10. Garniture hygiénique selon l'une des revendications 1 à 9, caractérisée en ce que la couverture inférieure (4) imperméable aux liquides est pourvue d'une bande (23) imprimée, s'étendant dans la direction longitudinale, revêtue d'une encre d'impression de préférence fortement hydrophobe, au moins dans la zone entourant les deux faces latérales (6 ; 6') du corps absorbant (2), sur le côté intérieur.

11. Garniture hygiénique selon l'une des revendications 1, 4 ou 6 à 10, caractérisée en ce que des volets de fixation, ayant une forme (25) découpée en une seule pièce ou en deux parties, sont collés sur la face inférieure (24), opposée au corps, de la couverture inférieure (4).
